Europäisches Patentamt

European Patent Office

Office européen des brevets

(11) Veröffentlichungsnummer: **0 332 937**
**A1**

# (12) EUROPÄISCHE PATENTANMELDUNG

(21) Anmeldenummer: 89103583.4

(22) Anmeldetag: 01.03.89

(51) Int. Cl.⁴: **A61B 6/00**

(30) Priorität: 14.03.88 DE 8803431 U

(43) Veröffentlichungstag der Anmeldung:
20.09.89 Patentblatt 89/38

(84) Benannte Vertragsstaaten:
**BE DE FR**

(71) Anmelder: **Siemens Aktiengesellschaft
Wittelsbacherplatz 2
D-8000 München 2(DE)**

(72) Erfinder: **Hahn, Alfred, Dipl.-Ing.
Bunsenstrasse 64
D-8520 Erlangen(DE)**

(54) **Röntgenuntersuchungseinrichtung mit zwei Bildaufnahmeeinheiten.**

(57) Bei einer Röntgenuntersuchungseinrichtung mit zwei Bildaufnahmeeinheiten (6, 7, 8; 12, 13, 18) soll ein kompakter Aufbau ohne Deckenlagerung von Komponenten geschaffen werden.

Die beiden Bildaufnahmeeinheiten (6, 7, 8; 12, 13, 18) sind an einem gemeinsamen Sockel (1) um eine gemeinsame horizontale Achse (4) schwenkbar gelagert. Der Träger (6) der ersten Bildaufnahmeeinheit (6, 7, 8) ist dabei direkt und der Träger (18) der zweiten Bildaufnahmeeinheit (12, 13, 18) ist über einen Zwischenträger (14) mit dem Sockel (1) verbunden. Auf diese Weise sind die beiden Bildaufnahmeeinheiten (6, 7, 8; 12, 13, 18) in zwei Ebenen verstellbar.

FIG 1

EP 0 332 937 A1

## Röntgenuntersuchungseinrichtung mit zwei Bildaufnahmeeinheiten

Die Erfindung betrifft eine Röntgenuntersuchungseinrichtung mit zwei Bildaufnahmeeinheiten, bestehend aus jeweils einem Träger mit einem Röntgenstrahler und einem Bildempfänger an seinen Enden, welche individuell einstellbar sind.

Es ist eine Röntgenuntersuchungseinrichtung dieser Art bekannt, bei der die eine Bildaufnahmeeinheit an einem Sockel und die zweite Bildaufnahmeeinheit an einem Deckenstativ schwenkbar gelagert ist. Der Patiententisch ist ebenfalls an der Decke des Untersuchungsraumes aufgehängt. Die kombinierte Decken- und Bodenmontage bedeutet einen erheblichen konstruktiven Aufwand. Ferner ist die Einstellmöglichkeit für Hilfsgeräte, wie z.B. einen Monitor, durch das Deckenstativ für die zweite Bildaufnahmeeinheit begrenzt.

Der Erfindung liegt die Aufgabe zugrunde, eine Röntgenuntersuchungseinrichtung der eingangs genannten Art so auszubilden, daß sich gegenüber dem Stand der Technik ein kompakterer Aufbau mit verbesserten Einstellmöglichkeiten für Hilfsgeräte ergibt.

Diese Aufgabe ist erfindungsgemäß dadurch gelöst, daß die beiden Bildaufnahmeeinheiten an einem gemeinsamen Sockel um eine gemeinsame horizontale Achse schwenkbar gelagert sind, wobei der Träger der ersten Bildaufnahmeeinheit direkt und der Träger der zweiten Bildaufnahmeeinheit über einen Zwischenträger mit dem Sockel verbunden ist, derart, daß die beiden Bildaufnahmeeinheiten in zwei Ebenen verstellbar sind. Durch die unmittelbare Lagerung des Trägers der ersten Bildaufnahmeeinheit am Sockel und die Verwendung eines Zwischenträgers zur Lagerung der zweiten Bildaufnahmeeinheit am Sockel ist es möglich, beide Bildaufnahmeeinheiten in der Weise in zwei zueinander senkrechten Ebenen zu verstellen, daß die Zentralstrahlen der beiden Bildaufnahmeeinheiten bei deren Verstellung in der jeweiligen Ebene bewegt werden. Der Zwischenträger und ein Halter des Trägers der ersten Bildaufnahmeeinheit sitzen dabei schwenkbar auf derselben Achse. Es ergibt sich ein kompakter Aufbau mit einem einzigen Sockel für beide Bildaufnahmeeinheiten.

Eine besonders zweckmäßige Weiterbildung der Erfindung besteht darin, daß beide Bildaufnahmeeinheiten an einem höhenverstellbaren Stativ gelagert sind, das Bestandteil des Sockels ist. Auf diese Weise ist es möglich, das gemeinsame Isozentrum beider Bildaufnahmeeinheiten, das im Schnittpunkt der Schwenkachse mit den Zentralstrahlen der Bildaufnahmeeinheiten liegt, in der Höhe zu verstellen.

Bei der erfindungsgemäßen Röntgenuntersuchungseinrichtung ist eine Verstellung des Trägers der zweiten Bildaufnahmeeinheit in Umfangsrichtung zur Änderung der Einstrahlrichtung nicht erforderlich, da diese Einstrahlrichtung durch Verschwenken der zweiten Bildaufnahmeeinheit um die gemeinsame Schwenkachse verändert werden kann.

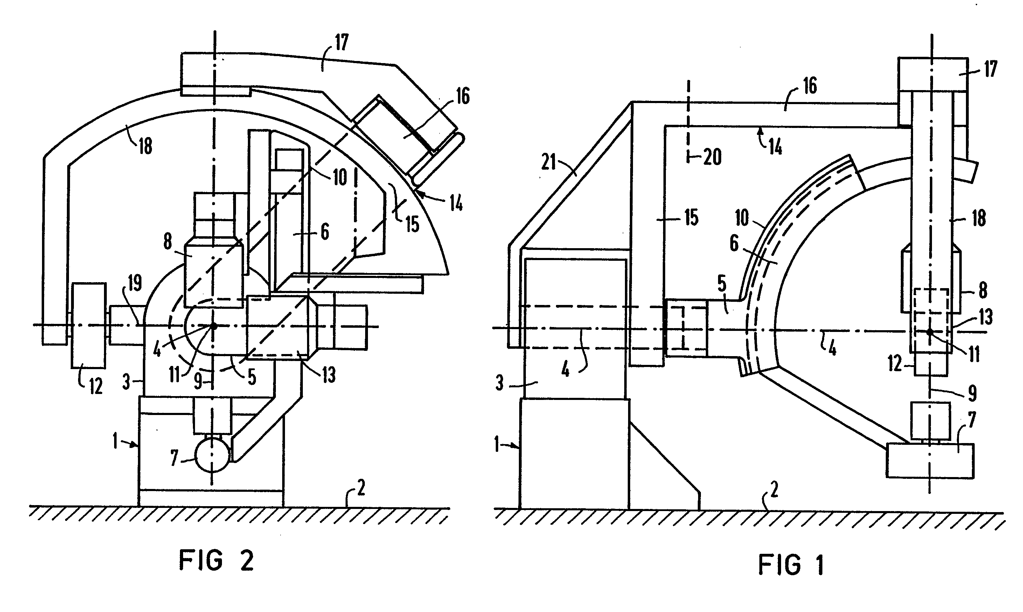

Die Erfindung ist nachfolgend anhand eines in den Fig. 1 und 2 in zwei verschiedenen Ansichten dargestellten Ausführungsbeispieles näher erläutert.

In der Zeichnung ist ein Sockel 1 dargestellt, der sich auf dem Fußboden 2 abstützt und ein höhenverstellbares Stativ 3 aufweist. Am Stativ 3 ist eine Achse 4 vorgesehen, um die ein Ansatz 5 eines Halters 10 für einen C-förmigen Träger 6 schwenkbar gelagert ist. Der Träger 6 trägt an seinen Enden zueinander ausgerichtet einen Röntgenstrahler 7 und einen Röntgenbildverstärker 8. Der Röntgenbildverstärker 8 ist in Richtung des Zentralstrahles 9 einstellbar mit dem Träger 6 verbunden. Der Zentralstrahl 9 trifft dabei im Zentrum des Eingangsleuchtschirmes des Röntgenbildverstärkers 8 auf. Der Träger 6 ist längs seines Umfanges im Halter 10 verstellbar, so daß Schrägdurchleuchtungen möglich sind. Der Zentralstrahl 9 schneidet die Achse 4 im Isozentrum 11.

Um die horizontale Achse 4 ist außer der Bildaufnahmeeinheit 6, 7, 8 eine zweite Bildaufnahmeeinheit mit einem Röntgenstrahler 12 und einem Röntgenbildverstärker 13 mit Hilfe eines L-förmigen Zwischenträgers 14 schwenkbar gelagert. Der Zwischenträger 14 ist mit dem Ende seines vertikalen Schenkels 15 schwenkbar auf der Achse 4 gelagert und trägt an seinem freien, horizontalen Schwenkel 16 einen Arm 17. Der Arm 17 trägt an seinem freien Ende einen U-förmigen Träger 18, an dessen Enden der Röntgenstrahler 12 und der Röntgenbildverstärker 13 befestigt sind. Der Träger 18 ist mit dem Ende des Armes 17 um eine Achse drehbar verbunden, die den Zentralstrahl 19 des Röntgenstrahlers 12 schneidet und in den Fig. 1 und 2 mit dem Zentralstrahl 9 zusammenfällt.

Der Arm 17 ist an seinem mit dem Schenkel 16 des Zwischenträgers 14 verbundenen Ende als Wagen ausgebildet und damit auf dem Schenkel 16 in dessen Längsrichtung verfahrbar. Dadurch kann die Bildaufnahmeeinheit 12, 13, 18 in eine Parkstellung 20 nahe dem Schenkel 15 und damit dem Sockel 1 verfahren werden.

Die Bildaufnahmeeinheit 6, 7, 8 ist durch Drehung des Halters 10 um die Achse 4 so einstellbar, daß sich der Zentralstrahl 9 in einer ersten Ebene bewegt. Die Lage dieser Ebene im Raum ist durch Verstellung des Trägers 6 gegenüber dem Halter 10 einstellbar.

Die Bildaufnahmeeinheit 12, 13, 18 ist durch Schwenken des Zwischenträgers 14 um die Achse 4 einstellbar, wobei sich der Zentralstrahl 19 in einer Ebene bewegt. Die Lage dieser Ebene im Raum ist durch Verschwenken des Trägers 18 gegenüber dem Arm 17 einstellbar. Beide Ebenen schneiden sich. In jeder Stellung der Bildaufnahmeeinheiten 6 bis 8 bzw. 12, 13, 18 schneiden sich die Zentralstrahlen 9, 19 im Isozentrum 11. Das Isozentrum 11 ist in der Höhe verstellbar, indem das Stativ 3 in seiner Höhe verstellt wird. Dadurch werden beide Bildaufnahmeeinheiten 6, 7, 8 bzw. 12, 13, 18 gemeinsam in ihrer Höhe eingestellt.

Der Zwischenträger 14 ist mit Hilfe einer Stütze 21 auf der Achse 4 am hinteren Ende des Sockels 1 abgestützt, so daß sich eine stabile Halterung ergibt.

Aus den Fig. geht hervor, daß sich der Zwischenträger 14 mit seinem freien Schenkel 16 über die Bildaufnahmeeinheit 6, 7, 8 parallel zur Schwenkachse 4 erstreckt und daß der Arm 17 rechtwinklig zum freien Schenkel 16 verläuft. Dadurch behindern sich die beiden Bildaufnahmeeinheiten 6, 7, 8; 12, 13, 18 bei ihrer Verstellung nicht gegenseitig.

Die Fig. zeigen eine kompakte Röntgenuntersuchungseinrichtung für die Untersuchung eines Patienten in zwei sich schneidenden Ebenen mit zwei Bildaufnahmeeinheiten. Eine Deckenaufhängung ist dabei für keine der Bildaufnahmeeinheiten erforderlich.

**Ansprüche**

1. Röntgenuntersuchungseinrichtung mit zwei Bildaufnahmeeinheiten (6, 7, 8; 12, 13, 18), bestehend aus jeweils einem Träger (6, 18) mit einem Röntgenstrahler (7, 12) und einem Bildempfänger (8, 13) an seinen Enden, welche individuell einstellbar sind, **dadurch gekennzeichnet,** daß die beiden Bildaufnahmeeinheiten (6, 7, 8; 12, 13, 18) an einem gemeinsamen Sockel (1) um eine gemeinsame horizontale Achse (4) schwenkbar gelagert sind, wobei der Träger (6) der ersten Bildaufnahmeeinheit (6, 7, 8) direkt und der Träger (18) der zweiten Bildaufnahmeeinheit (12, 13, 18) über einen Zwischenträger (14) mit dem Sockel (1) verbunden ist, derart, daß die beiden Bildaufnahmeeinheiten (6, 7, 8; 12, 13, 18) in zwei Ebenen verstellbar sind.

2. Röntgenuntersuchungseinrichtung nach Anspruch 1, **dadurch gekennzeichnet,** daß der Zwischenträger (14) L-förmig ausgebildet ist, mit seinem einen Ende auf der gemeinsamen Achse (4) schwenkbar gelagert und an seinem anderen, über die erste Bildaufnahmeeinheit (6, 7, 8) parallel zur Schwenkachse (4) ragenden Ende den Träger (18) der zweiten Aufnahmeeinheit (12, 13, 18) trägt.

3. Röntgenuntersuchungseinrichtung nach Anspruch 2, **dadurch gekennzeichnet,** daß der Träger (6) der ersten Bildaufnahmeeinheit (6, 7, 8) mit einem Halter (5) verbunden ist, der um die horizontale Achse (4) schwenkbar ist.

4. Röntgenuntersuchungseinrichtung nach Anspruch 2 oder 3, **dadurch gekennzeichnet,** daß die zweite Bildaufnahmeeinheit (12, 13, 18) an einem als Wagen ausgebildeten Arm (17) befestigt ist, der sich rechtwinklig zum freien Schenkel (16) des Zwischenträgers (14) erstreckt.

5. Röntgenuntersuchungseinrichtung nach Anspruch 4, **dadurch gekennzeichnet,** daß die zweite Bildaufnahmeeinheit (12, 13, 18) am Arm (17) um eine senkrecht zu ihrem Zentralstrahl (19) verlaufende Achse (9) drehbar mit dem Arm (17) verbunden ist.

6. Röntgenuntersuchungseinrichtung nach einem Ansprüche 1 bis 5, **dadurch gekennzeichnet,** daß beide Bildaufnahmeeinheiten (6, 7, 8; 12, 13, 18) an einem höhenverstellbaren Stativ (3) gelagert sind, das Bestandteil des Sockels (1) ist.

FIG 1

FIG 2

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| Y | GB-A-2 120 060 (J.K. GRADY) * Zusammenfassung; Seite 1, Zeile 104 - Seite 2, Zeile 14; Seite 2, Zeilen 30-48,90-105; Figuren 1-9 * | 1,3,5 | A 61 B 6/00 |
| A | --- | 4 | |
| Y | BIOMEDIZINISCHE TECHNIK, Band 29, Nr. 10, Oktober 1984, Seiten 261-266, Berlin, DE; H. WOLLSCHLÄGER et al.: "Biplane multidirectional angiocardiography: exact orthogonal positioning of the X-ray systems (laterally mounted C-arm-L-arm-systems) * Seiten 261-263; Zusammenfassung; Figur 1 * | 1,3,5 | |
| A | IDEM --- | 2 | |
| A | EP-A-0 069 607 (THOMSON-CSF) * Zusammenfassung; Seite 6, Zeilen 12-32; Seite 7, Zeilen 4-13; Seite 7, Zeile 31 - Seite 8, Zeile 13; Seite 9, Zeilen 4-32; Seite 10, Zeilen 10-22; Figur 2 * | 1,3,4,6 | RECHERCHIERTE SACHGEBIETE (Int. Cl.4) |
| A | --- FR-A-2 117 344 (SIEMENS AG) ----- | | A 61 B |

Der vorliegende Recherchenbericht wurde für alle Patentansprüche erstellt

| Recherchenort | Abschlußdatum der Recherche | Prüfer |
|---|---|---|
| DEN HAAG | 09-06-1989 | RIEB K.D. |

**KATEGORIE DER GENANNTEN DOKUMENTE**

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument
.......................................................................
& : Mitglied der gleichen Patentfamilie, übereinstimmendes Dokument